(19) Europäisches Patentamt
European Patent Office
Office européen des brevets

(11) Publication number: **0 139 201**
**B1**

(12) **EUROPEAN PATENT SPECIFICATION**

(45) Date of publication of patent specification: **07.12.88**

(51) Int. Cl.$^4$: **G 01 N 33/534,**
G 01 N 33/53, G 01 N 33/68,
C 07 K 7/06, C 07 K 5/10

(21) Application number: **84110703.0**

(22) Date of filing: **07.09.84**

(54) **Diagnostic method and kit.**

(30) Priority: **09.09.83 GB 8324167**

(43) Date of publication of application:
**02.05.85 Bulletin 85/18**

(45) Publication of the grant of the patent:
**07.12.88 Bulletin 88/49**

(84) Designated Contracting States:
**DE FR GB IT SE**

(56) References cited:
**EP-A-0 070 478**
**DD-A- 157 095**
**GB-A-2 072 192**
**US-A-3 983 099**

**CHEMICAL ABSTRACTS, vol. 93, no. 7, August 18, 1980, Columbus, Ohio, USA, WEI E.T. et al. "Cardiuvascular effects of peptides related to the enkephalines and beta-casomorphin", abstract no. 61 992s**

(73) Proprietor: **THE WELLCOME FOUNDATION LIMITED**
**183-193 Euston Road**
**London NW1 2BP (GB)**

(72) Inventor: **Chang, Kwen-Jen**
**2476 Foxwood Drive**
**Chapel Hill North Carolina 27514 (US)**

(74) Representative: **Sandmair, Kurt, Dr. et al**
**Patentanwälte Schwabe, Sandmair, Marx**
**Stuntzstrasse 16**
**D-8000 München 80 (DE)**

**EP 0 139 201 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

The present invention relates to a method for the identification of human infants at risk from the sudden infant death syndrome, and to compounds and a diagnostic kit of value for the performance thereof.

The sudden infant death syndrome (SIDS), alternatively called cot death or crib death, has been defined as "the sudden death of any infant or young child which is unexpected by history and in which a thorough post mortem examination fails to demonstrate an adequate cause for death". It is recognized as a distinct clinical entity that results in about 1500 deaths a year in the United Kingdom and 8000 deaths a year in the United States of America: in one study the syndrome accounted for 45% of postperinatal infant deaths and 20% of all deaths from 8 days to 14 years of age (for reviews see Valdes-Dapena, M.A.: *Pediatrics 66*: 597, 1980 and Guntheroth, W.G.: *Am. Heart J. 93*: 784, 1977).

Despite increasing research into the syndrome its pathogenesis remains obscure and there is no satisfactory method for the identification (prediction) of infants at risk. The problem at hand is identified thus by Valdes-Dapena (*loc. cit.*):

"Ideally, the physician should be able to determine, upon the basis of physical examinations and certain tests, whether or not a newborn baby is apt to die of sudden infant death syndrome. Unfortunately, although some progress has been made toward that end within the past decade, that day has not yet arrived . . . Thus, at this time, there is no reliable screening mechanism for sudden infant death syndrome; it remains an entity, the occurrence of which, for any specific infant, cannot be predicted with any degree of certainty".

The present invention provides a method for the identification of such at-risk infants based on a determination of the level (concentration) in their body fluids, preferably blood, of either or both of the peptides morphiceptin (I) and β-casomorphin (II)

H-Tyr-Pro-Phe-Pro-$NH_2$ (I)

H-Tyr-Pro-Phe-Pro-Gly-Pro-Ile-OH (II)

(all chiral amino acid radicals are in the L-configuration).

It is envisaged that such a determination procedure will form a part of the routine perinatal assessment of all infants with those shown to have an abnormally high blood level of either or both of the said peptides being regarded as at risk from the syndrome. There is therefore enabled the possibility of monitoring thus-selected infants in an attempt to avert their falling victim to SIDS.

In one aspect therefore there is now provided a method for ascertaining whether a human infant is at risk from the sudden infant death syndrome comprising the steps of

a) measuring the concentration in a blood sample from the infant of at least one of morphiceptin and β-casomorphin; and

b) determining the risk to the infant using the blood concentration measurement(s).

In a preferred aspect the concentration of β-casomorphin is measured.

Measurement of the peptide concentration(s) in the blood sample (step(a)) may be effected using any conventional technique however, for general convenience, immunoassay is preferred. When an immunoassay procedure is employed the known peptide(s) may be labelled in any manner enabling determination of the amount bound; radiolabelling (radioimmunoassay) is particularly convenient using for example a compound labelled with $^3H$ or, preferably, a γ-emitting isotope such as $^{125}I$. Conveniently the assay is carried out upon a morphiceptin/β-casomorphin-containing fraction prepared from the blood sample as originally withdrawn, for example plasma, serum or a fraction thereof.

Morphiceptin and β-casomorphin may be labelled with $^{125}I$ by the chloramine T method standard in the art to provide respectively the compounds (Ia) and (IIa)

H-($^{125}I$)iodo-Tyr-Pro-Phe-Pro-$NH_2$ (Ia)

H-($^{125}I$)iodo-Tyr-Pro-Phe-Pro-Gly-Pro-Ile-OH (IIa)

A diagnostic kit of value for the performance of the method described herein comprises

a) at least one of morphiceptin and β-casomorphin labelled in a manner enabling determination of the amount thereof bound in an immunoassay procedure,

b) (complementary to a)) at least one of antibodies against morphiceptin and antibodies against β-casomorphin, and

c) (complementary to a)) a standard of at least one of morphiceptin and β-casomorphin,

the materials being held in separate sealed vessels.

In a preferred aspect such a kit comprises, as a), at least one of $^{125}I$-labelled morphiceptin and $^{125}I$-labelled β-casomorphin; in a more preferred aspect a) is $^{125}I$-labelled β-casomorphin.

Conveniently the said kit also comprises at least one of

d) a sealed vessel containing a buffer solution, and

e) a sealed vessel containing a protease inhibitor, for example bacitracin.

One or more of the above-indicated materials, may if desired, be held in a freeze-dried (lyophilized) condition until required for use.

Blood sample collection and fractionation

1. Draw 1 ml blood into tubes containing EDTA
2. Place the tubes in a 4°C ice bucket
3. Centrifuge to separate the cells from the plasma
4. Mix 0.5 ml plasma with 0.5 ml cold 10% trichloroacetic acid
5. Centrifuge to separate the resulting precipitate from the supernatant
6. Prepare a reverse-phase C-18 Sep-Pack® cartridge; wet the cartridge with methanol, then 0.1 M acetic acid
7. Apply the supernatant from 5. to the prewetted C-18 Sep-pack cartridge
8. Wash the cartridge with 30 ml 1 M acetic acid
9. Elute peptides from the cartridge with 3 ml acetic acid-methanol (1:17)
10. Dry the eluate under nitrogen
11. Lyophilize the residue
12. Dissolve the residue in 0.20 M phosphate buffer (pH 7.4)
13. Assay for morphiceptin-like and β-casomorphin-like activities

Radioimmunoassay for morphiceptin-like and β-casomorphin-like activities

1. Synthetic morphiceptin and β-casomorphin were prepared using solid-phase techniques.

2. Antisera against morphiceptin and β-casomorphin were raised in male New Zealand white rabbits. The respective peptide was conjugated to bovine serum albumin (Pentex® 5×crystallized) by 1% glutaraldehyde in 0.20 M sodium phosphate buffer (pH 7.4) for 15 min at room temperature. The conjugate was separated from unreacted peptide by gel filtration chromatography on a Sephadex® G-25 column. The void volume fraction containing conjugate (200 μg peptide) was then emulsified with an equal volume of Freund's complete adjuvant and injected into rabbits subcutaneously on both sides of the body and intramuscularly on both hind paws. The rabbits were boosted every three weeks with the conjugate (about 50 μg peptide) emulsified with Freund's incomplete adjuvant.

3. Radioimmunoassay (RIA) using protein A-containing Staphylococcus as solid phase support (ref. 1) was employed. Specific antiserum (0.5 ml) was incubated with washed Staphylococcus in a 50 mM Tris. HCl buffer (pH 7.7) containing 0.5 mM EDTA and 0.5% Triton® X-100 for 3 hr to absorb most IgG. The IgG-absorbed Staphylococcus was then washed twice with the same buffer. The final pellet was suspended in 5 times the original serum volume and stored at 4°C.

4. $^{125}$I-labelled morphiceptin and β-casomorphin were prepared by the chloramine T method (ref. 2). An aliquot (5—10 μl, $11{,}1\times10^7$ Bg (3 mCi) of carrier free and freshly prepared Na $^{125}$I (Union Carbide) was added to 100 μl of 0.25 M sodium phosphate buffer (pH 7.4) containing 1.5 nmol of peptide. The iodination was initiated by adding 20 μl of 0.5 mg/ml Chloramine T and terminated 20 seconds later by adding 20 μl of 1 mg/ml sodium metabisulfite. 100 μl of 0.1% bovine serum albumin containing Tris. HCl (50 mM) buffer was added 30 s later. The whole reaction mixture was applied to Biol-Gel®-P-2 (12 ml) pre-equilibrated with Tris. HCl—0.1% bovine serum albumin buffer solution. The iodinated peptide peaks (8—9 ml fractions for β-casomorphin, 10—11 ml fractions for morphiceptin) were collected.

5. RIA was carried out in 0.20 ml of sodium phosphate buffer (pH 7.4) which is 0.5 mM with respect to EDTA and contains 1% Triton X-100 for 18 h. at 4°C with about 10,000 cpm of $^{125}$I-labelled peptide. At the end of the incubation, 1 ml of the same cold buffer was added to each tube and the mixture centrifuged for 20 min at 3500 rpm in a Sorvall RC-5 (rotor GSP-25). The supernatant was aspirated. The tubes containing Staphylococcus were counted in a γ-counter at 75% efficiency. The nonspecific binding (less than 5%) was determined by the presence of an excess of unlabelled peptide (10 μM).

6. The amounts of morphiceptin-like and β-casomorphin-like activities in unknown samples were determined from standard curves obtained with the synthetic peptides.

Ref. 1: O'Keefe, E. and Bennett, V. (1980) *J. Biol. Chem. 255*, 561—568
Ref. 2: Hunter, W. M., *et al.* (1962) *Nature 194*, 495—496

Alternative radioimmunoassay procedure

Reagents:

1. *Buffer*. All dilutions made in 154 mM NaCl, 25 mM $Na_2HPO_4$ pH 7.5, 0.1% Triton X-100 (trade name).

2. *Bacitracin*. 25 mg/ml in buffer, to give 0.5 mg/ml final concentration in the assay. Store at 4°C.

3. *Solid Phase Immunoadsorbent*. Donkey anti-rabbit IgG immobilized on beaded cellulose (Wellcome Reagents Anti-Rabbit Sac-Cel). Wash and resuspend to original volume in buffer.

4. *Antiserum* (rabbit). Prepare a working dilution of 1:200 in buffer. Final dilution in assay to be 1:10,000.

5. *Radiolabel*. Prepare a working concentration of radiolabel of $5—7\times10^6$ cpm/ml to give approximately 35,000 cpm per assay tube.

6. *Standards*. Prepare stock solutions of each peptide at 1.0 mM in buffer. Store at −20°C. Prepare initial

working dilutions of 1.0 (for β-casomorphin) or 2.0 (for morphiceptin) micromolar in buffer. Dilute this by factors of 3 to prepare 6 standards ranging from 5—1000 nM (10—2000) to give 0.02—5.0 pmol/tube (0.04—10.0) in the assay.

7. *NSB Standard.* Use 0.1 mM peptide in buffer to give 500 pmol/tube in the assay.

Protocol:

1. All reagents, procedures are performed at 4°C.
2. All tubes are adjusted to a final volume of 250 microlitres in the assay.
3. Reagents 1 and 5 may be combined prior to addition for convenience, as may reagents 3 and 4.
4. The assay is initiated with the addition of the antibody-gel reagent. Incubation ranges from 3—4 hours at 4°C.
5. The assay is terminated with the addition of 4 ml of ice-cold buffer to all tubes (except TA).
6. Centrifuge samples 15 minutes at 3000 rpm in Sorval RC 3 at 4°C.
7. Aspirate and count pellets.

| Condition | Buffer | Bacit. | Label | Stds | NSB | Unkn | Antib. | Gel |
|---|---|---|---|---|---|---|---|---|
| Total added (TA) | — | — | 5 | — | — | — | — | — |
| Total bound (TB) | 210 | 5 | 5 | — | — | — | 5 | 25 |
| Non-specific (NSB) | 205 | 5 | 5 | — | 5 | — | 5 | 25 |
| Standard curve | 205 | 5 | 5 | 5 | — | — | 5 | 25 |
| Unknowns (serum) | var | 5 | 5 | — | — | var | 5 | 25 |

(all volumes are microlitres)

Sample data: (β-casomorphin)

| Condition | cpm | Average | -NSB | %B/B$_0$ | pmol/tube |
|---|---|---|---|---|---|
| Total added | 35740<br>35604 | 35672 | — | — | — |
| Total bound | 2350<br>2402 | 2376 | 2039 | 100.0 | — |
| Nonspecific | 330<br>344 | 337 | — | — | — |
| Standard (a) | 560<br>638 | 599 | 262 | 12.8 | 5.0 |
| (b) | 899<br>947 | 923 | 586 | 28.7 | 1.7 |
| (c) | 1431<br>1493 | 1462 | 1125 | 55.2 | 0.6 |
| (d) | 1850<br>2042 | 1946 | 1609 | 78.9 | 0.2 |
| (e) | 2110<br>2412 | 2261 | 1924 | 94.4 | 0.06 |
| (f) | 2300<br>2396 | 2348 | 2011 | 98.6 | 0.02 |
| Unknown (a) | 1024<br>879 | 952 | 615 | 25.9 | 2.1 |
| (b) | 1650<br>1571 | 1611 | 1274 | 53.6 | 0.67 |
| (c) | 2075<br>1963 | 2019 | 1682 | 70.8 | 0.32 |

**Claims**

1. A method for ascertaining whether a human infant is at risk from the sudden infant death syndrome comprising the steps of

a) measuring the concentration in a blood sample from the infant of at least one of morphiceptin and β-casomorphin; and

b) determining the risk to the infant using the blood concentration measurement(s).

2. The method of claim 1 wherein the concentration of β-casomorphin is measured.

3. The method of either of claims 1 and 2 wherein the concentration is measured by immunoassay.

4. The method of either of claims 1 and 2 wherein the concentration is measured by radioimmunoassay.

5. The method of either of claims 1 and 2 wherein the concentration is measured by radioimmunoassay using a compound labelled with $^{125}I$.

6. The method of any of claims 1 to 5 wherein the concentration in plasma prepared from the blood sample is measured.

7. The method of any of claims 1 to 5 wherein the concentration in serum prepared from the blood sample is measured.

8. As a mercantile unit, a kit comprising

a) at least one of morphiceptin and β-casomorphin labelled in a manner enabling determination of the amount thereof bound in an immunoassay procedure,

b) (complementary to a)) at least one of antibodies against morphiceptin and antibodies against β-casomorphin, and

c) (complementary to a)) a standard of at least one of morphiceptin and β-casomorphin,

the materials being held in separate sealed vessels.

9. The kit of claim 8 comprising, as a), at least one of radiolabelled morphiceptin and radiolabelled β-casomorphin.

10. The kit of claim 8 comprising, as a), at least one of $^{125}I$-labelled morphiceptin and $^{125}I$-labelled β-casomorphin.

11. The kit of any of claims 8 to 10 comprising, as a), labelled β-casomorphin.

12. The kit of claim 8 comprising

a) $^{125}I$-labelled β-casomorphin,

b) antibodies against β-casomorphin, and

c) a standard for β-casomorphin.

13. The kit of claim 8 additionally comprising a sealed vessel containing a buffer solution.

14. The kit of claim 8 additionally comprising a sealed vessel containing a protease inhibitor.

15. A compound selected from

morphiceptin labelled in a manner enabling determination of the amount thereof found in an immunoassay procedure, and

β-casomorphin labelled in a manner enabling determination of the amount thereof bound in an immunoassay procedure.

16. A compound selected from

$$H\text{-}(^{125}I)iodo\text{-}Tyr\text{-}Pro\text{-}Phe\text{-}Pro\text{-}NH_2$$

and

$$H\text{-}(^{125}I)iodo\text{-}Tyr\text{-}Pro\text{-}Phe\text{-}Pro\text{-}Gly\text{-}Pro\text{-}Ile\text{-}OH.$$

**Patentansprüche**

1. Verfahren zur Feststellung, ob bei einem menschlichen Säugling das Risiko des plötzlichen Kindstodes-Syndroms besteht, bei dem man

a) die Konzentration mindestens eines von Morphiceptin und ß-Casomorphin in einer Blutprobe des Säuglings bestimmt, und

b) das Risiko für den Säugling unter Verwendung der Blutkonzentrations-Messung(en) bestimmt.

2. Verfahren nach Anspruch 1, bei dem man die Konzentration von ß-Casomorphin bestimmt.

3. Verfahren nach Anspruch 1 oder 2, bei dem man die Konzentration mittels Immunoassay bestimmt.

4. Verfahren nach Anspruch 1 oder 2, bei dem man die Konzentration mittels Radioimmunoassay bestimmt.

5. Verfahren nach einem der Ansprüche 1 oder 2, bei dem man die Konzentration mittels Radioimmunoassay unter Verwendung einer mit $^{125}$Iod markierten Verbindung bestimmt.

6. Verfahren nach einem der Ansprüche 1 bis 5, bei dem man die Konzentration in aus der Blutprobe hergestelltem Plasma bestimmt.

7. Verfahren nach einem der Ansprüche 1 bis 5, bei dem man die Konzentration in aus der Blutprobe hergestelltem Serum bestimmt.

8. Handelsprodukt in Form eines Kits, enthaltend

a) mindestens eines von Morphiceptin und ß-Casomorphin, das in einer Art und Weise markiert ist, die die Bestimmung der in einem Immunoassay - Verfahren gebundenen Menge erlaubt,

b) (ergänzend zu a)) mindestens einen der Antikörper gegen Morphiceptin und Antikörper gegen ß-Casomorphin, und

c) (ergänzend zu a)) einen Standard von mindestens einem von Morphiceptin und ß-Casomorphin, wobei die Materialien in getrennten, verschlossen Gefäßen vorliegen.

9. Kit nach Anspruch 8, enthaltend als a) mindestens eines von radioaktivmarkierten Morphiceptin und radioaktivmarkierten ß-Casomorphin.

10. Kit nach Anspruch 8, enthaltend als a) mindestens eines von $^{125}$Iod markiertem Morphiceptin und $^{125}$Iod markiertem ß-Casomorphin.

11. Kit nach einem der Ansprüche 8 bis 10, enthaltend als a) markiertes ß-Casomorphin.

12. Kit nach Anspruch 8, enthaltend

a) $^{125}$Iod markiertes ß-Casomorphin,

b) Antikörper gegen ß-Casomorphin, und

c) einen Standard von ß-Casomorphin.

13. Kit nach Anspruch 8, der zusätzlich ein verschlossenes Gefäß, das eine Pufferlösung enthält, beinhaltet.

14. Kit nach Anspruch 8, der zusätzlich ein verschlossenes Gefäß, das einen Protease-Inhibitor enthält, beinhaltet.

15. Verbindung, ausgewählt aus Morphiceptin, markiert in einer Weise, die die Bestimmung der in einem Immunoassay - Verfahren gebundenen Menge davon ermöglicht, und

ß-Casomorphin, markiert in einer Weise, die die Bestimmung der in einem Immunoassay-Verfahren gebundenen Menge davon ermöglicht.

16. Verbindung ausgewählt aus

$$H\text{-}(^{125}I)iodo\text{-}Tyr\text{-}Pro\text{-}Phe\text{-}pro\text{-}NH_2$$

und

$$H\text{-}(^{125}I)iodo\text{-}Tyr\text{-}Pro\text{-}Phe\text{-}pro\text{-}Gly\text{-}Pro\text{-}Ile\text{-}OH.$$

**Revendications**

1. Une méthode pour déterminer si un nouveauné présente le risque du syndrome de mort subite du nourrisson, méthode selon laquelle:

a) on mesure sur un prélèvement de sang le taux de morphiceptine ou de β-casamorphine ou les deux; et

b) on détermine le risque d'après ce taux.

2. La méthode selon la revendication 1 dans laquelle on mesure le taux de β-casamorphine.

3. La méthode selon la revendication 1 ou 2 dans laquelle on mesure les taux par un titrage immunologique.

4. La méthode selon la revendication 1 ou 2 dans laquelle on mesure les taux par un titrage radio-immunologique.

5. La méthode selon la revendication 1 ou 2 dans laquelle on mesure les taux par un titrage radio-immunologique avec un composé marqué à l'iode 125.

6. La méthode selon l'une quelconque des revendications 1 à 5 dans laquelle on mesure les taux sur le plasma de l'échantillon de sang.

7. La méthode selon l'une quelconque des revendications 1 à 5 dans laquelle on mesure les taux sur le sérum de l'échantillon de sang.

8. Une trousse (kit) qui comprend:

a) de la morphiceptine ou de la β-casamorphine ou les deux, qui sont marquées de façon que l'on puisse doser leurs quantités liées par un titrage immunologique,

b) (en complément de a)) des anticorps contre la morphiceptine ou la β-casamorphine ou contre les deux, et

c) (en complémente de a)) un étalon (titre) de morphiceptine ou de β-casamorphine ou les deux, ces matières étant conservées séparément en récipients scellés.

9. La trousse selon la revendication 8 qui comprend de la morphiceptine ou de la β-casamorphine ou les deux, marquées avec un isotope radioactif.

10. La trousse selon la revendication 8 qui comprend de la morphiceptine ou de la β-casamorphine ou les deux, marquées à l'iode 125.

11. La trousse selon l'une quelconque des revendications 8 à 10 qui comprend de la β-casamorphine marquée.

12. La trousse selon la revendication 8 qui comprend:

a) de la β-casamorphine marquée à l'iode 125,

b) des anticorps contre la β-casamorphine, et

c) un étalon (titre) de β-casamorphine.

13. La trousse selon la revendication 8 qui comprend en outre une solution tampon en récipient scellé.

14. La trousse selon la revendication 8 qui comprend en outre un inhibiteur de protéase en récipient scellé.

15. La morphiceptine marquée de façon à permettre le dosage de sa quantité liée par un titrage immunologique, et

la β-casamorphine marquée de manière à permettre le dosage de sa quantité liée par un titrage immunologique.

16. Les composés suivants:

$$H\text{-}(^{125}I)iodo\text{-}Tyr\text{-}Pro\text{-}Phe\text{-}Pro\text{-}NH_2$$

et

$$H\text{-}(^{125}I)iodo\text{-}Tyr\text{-}Pro\text{-}Phe\text{-}Pro\text{-}Gly\text{-}Pro\text{-}Ile\text{-}OH.$$